# EUROPÄISCHE PATENTSCHRIFT

(11) **EP 0 734 229 B1**
(45) Veröffentlichungstag und Bekanntmachung des Hinweises auf die Patenterteilung: **29.04.1998**
(21) Anmeldenummer: 95902748.3
(22) Anmeldetag: 12.12.1994
(51) Int. Cl.: A61B 17/02, A61B 19/00, A61F 13/00

(54) **VORRICHTUNG ZUR STIMULATION DER NEUBILDUNG VON GEWEBE BEI GROSSFLÄCHIGEN UND TIEFEN WUNDEN**
DEVICE FOR STIMULATING THE FORMATION OF NEW TISSUES IN EXTENSIVE AND DEEP WOUNDS
DISPOSITIF DE STIMULATION DE LA FORMATION DE TISSUS NOUVEAUX DANS DES PLAIES PROFONDES ET DE GRANDE EXTENSION

(30) Priorität: 13.12.1993 DE 4342457
(43) Veröffentlichungstag der Anmeldung: 02.10.1996
(73) Patentinhaber: Fleischmann, Wim, 89182 Bernstadt (DE)
(72) Erfinder: Fleischmann, Wim, 89182 Bernstadt (DE)
(74) Vertreter: Patentanwälte Westphal, Buchner, Mussgnug Neunert, Göhring
(86) Internationale Anmeldenummer: DE9401467
(87) Internationale Veröffentlichungsnummer: WO9516397

(56) Entgegenhaltungen:
- WO-A-93/09727
- DE-A- 4 103 070
- US-A- 3 735 765
- US-A- 5 263 971

## Beschreibung

Die Erfindung betrifft eine Vorrichtung zur Stimulation der Neubildung von Gewebe bei großflächigen Wunden und tiefen Gewebsdefekten gemäß dem Oberbegriff der unabhängigen Patentansprüche.

Großflächige, durch offene Frakturen oder äußere Einwirkung verursachte Wunden sind in besonderem Maße Krankheitskeimen ausgesetzt, da die tieferen Gewebeschichten bis hin zum verletzten Knochen der Umgebung exponiert sind.

Bereits eine Infektion durch Staphylo- oder Streptokokken kann für den Verletzten lebensbedrohlich sein. Die Kontaminierung der großflächigen Wunde durch Proteus, Pseudomonas oder Klepsiella führen in der Regel zu einem lebensbedrohlichen septischen Zustandsbild. Ferner wird der Heilungsprozeß durch die Nekrotisierung zerstörter Gewebeanteile belastet. Diese hochtoxischen Eiweißzerfallsprodukte werden verflüssigt als Wundsekret vom Organismus abgestoßen. Entsprechend dem Verletzungsgrad sind deshalb mehr oder weniger häufig für den Verletzten äußerst schmerzhafte Nachbehandlungen der Wunde notwendig.

Für die operative Versorgung von großflächigen Wunden wird in jüngster Zeit die Technik der Vakuumversiegelung angewandt (z. B. WO 93/09727). Hierbei wird nach erster operativer Versorgung der Wunde der Gewebedefekt oder Hautschnitt und etwaige Gewebetaschen mit Lagen von Polyvinylschaumstoff aufgefüllt, wobei in die benachbarten Schaumstofflagen sogenannte Redon-Drainagen direkt in das Schaumstoffmaterial eingebracht werden. Es ist dabei darauf zu achten, daß die Drainageperforationen nicht mit Weichteilen in Verbindung geraten, da sie sich bei Anlegen eines Vakuums sonst festsaugen und ihre Drainagefunktion verlieren würden. Die so eingebrachten Lagen von Polyvinylschaumstoff werden anschließend mit einer transparenten, wasserdampfpermeablen aber bakterienundurchlässigen Polyurethanfolie derart versiegelt, daß die Polyurethanfolie die Wunde sowie an die Wundränder angrenzende unverletzte Haut überdeckt. Nach Anschluß der Redon-Drainagen an ein Vakuumsystem wird das Wundsekret abgesaugt und gleichzeitig ein intensiver Kontakt zwischen Wunde und Schaumstoff bewirkt, der - wie die Erfahrung gezeigt hat - die Wundreinigung erheblich verbessert und die Neubildung von Granulationsgewebe fördert.

Es hat sich jedoch gezeigt, daß der auf dem Gewebe ruhende atmosphärische Druck die Durchblutung im Randbereich der Wunde verschlechtert und vielfach zu Muskelnekrosen und zur Devitalisierung von Geweberegionen führt. Im geschädigten Gewebe entstehen häufig Kammern, in denen sich toxische Eiweißzerfallsprodukte ansammeln, die zu einem lebensbedrohlichen septischen Zustand des Verletzten führen können. Die Genesung kann - wenn überhaupt - nur durch schwierige, den Organismus zusätzlich belastende operative Eingriffe, flankiert durch langwierige, kostenintensive und schmerzhafte Wundversorgung herbeigeführt werden.

Weiter ist es bekannt (z. B. US 4,896,680 und US 5,263,971), die Wundränder einer großflächigen Wunde mittels eines sogenannten Haut-Distraktors zusammenzuziehen, um die Wundränder miteinander vernähen zu können. Der Hautdistraktor greift mit zwei Distraktorsegmenten jeweils an einander gegenüberliegenden Wundrändern in die Cutis ein. Die Distraktorsegmente werden über eine Gewindespindel zusammengezogen. Das Zusammenziehen erfolgt in aufeinanderfolgenden Schritten, wobei die Haut bei jedem Schritt bis an die Grenze ihrer viskoelastischen Dehnbarkeit gestreckt wird. Der bekannte Hautdistraktor erzeugt Zugkräfte nur in der Ebene des Wundfeldes, um die Wundränder unter Ausnützung der viskoelastischen Eigenschaften der Haut zum Vernähen zusammenzuziehen. Eine Neubildung von Gewebe ist hierbei nicht möglich und nicht beabsichtigt.

Die Aufgabe der Erfindung besteht darin, die Behandlung des Gewebsdefektes durch Sicherstellung der Gewebevitaltität und Förderung der Gewebsneubildung zu verbessern, die bei großflächigen Wunden häufig auftretenden Komplikationen zu vermeiden und insbesondere Vorsorge zu treffen, daß das Zusammenwachsen des Coriums wesentlich verbessert wird.

Diese Aufgabe ist gemäß der Erfindung gelöst durch eine Vorrichtung nach den Ansprüchen 1 und 8.

Weitere Merkmale der Erfindung ergeben sich aus den Unteransprüchen.

Die Erfindung nutzt die Erkenntnis, daß eine im Wundbereich mit einer zur Ebene der Wundfläche senkrechten Komponente auf das Gewebe wirkende Zugspannung unter Gewebsneubildung zu einer kontinuierlichen Wundverkleinerung bei gleichzeitiger Ausbildung eines gleichmäßig strukturierten Granulationsrasens und Auffüllung des Gewebedefektes zu einer rascheren Wundverkleinerung führt. Dabei wird die Durchblutung des Gewebes erheblich verbessert, wodurch eine Verkürzung der Heilungsvorgänge erfolgt und vielfach auf Zusatzoperationen zum definitiven Wundverschluß verzichtet werden kann.

Bei einer Vakuumversiegelung der Wunde mit einer Kunststofffolie wird durch die Aufbringung von Zugkräften über die Kunststofffolie oder unter der Kunststofffolie angeordnete Schaumstofflagen auf den Gewebedefekt eine beschleunigte Gewebsregeneration bewirkt. Ferner bleibt auch bei einer langdauernden posttraumatischen und postoperativen Ödemphase die Gewebedurchblutung aufrechterhalten. Auch bleibt die geschützte Wunde über einen längeren Zeitraum für Sekundäreingriffe gut konditioniert. Die so behandelten großflächigen und tiefen Gewebsdefekte werden daher stärker entgiftet, besser durchblutet, wodurch die Mortalität erheblich gemindert wird.

Zum Zusammenwachsen der Cutis kann ein Hautdistraktor verwendet werden, der die Wundränder horizontal, d. h. in der Ebene der Wundfläche, zusammenzieht. Auf diesen Hautdistraktor wird zusätzlich eine zur Wundfläche senkrechte (vertikale) Kraftkomponente ausgeübt, um das Gewebe im Bereich der Wundränder zu entlasten und die Gewebsneubildung im Bereich der Cutis zu fördern. Im Gegensatz zu den bekannten Hautdistraktoren werden dabei die Wundränder nur sehr langsam (über einen Zeitraum von Tagen oder Wochen) zusammengezogen, so daß die Haut nicht viskoelastisch gedehnt wird, sondern die Wundränder durch Gewebsneubildung zusammenwachsen.

Das Zusammenwachsen der Cutis kann durch empirisch zu ermittelndes Einstellen der Distraktions- oder Zugkräfte sowohl horizontal auf die Wundränder als auch vertikal auf den Gewebsdefekt gezielt gesteuert werden, so daß auch bei großflächigen Gewebsdefekten die Narbenbildung klein bleibt und posttraumatische Folgeerscheinungen vermieden werden können.

Schließlich kann der schmerzhafte Verbandwechsel bei Anwendung des erfindungsgemäßen Verfahrens reduziert werden. Insgesamt tritt eine Verkürzung der Heilungsdauer ein.

Die Erfindung ist nachfolgend an Hand eines in der Zeichnung schematisch dargestellten Ausführungsbeispiels beschrieben. Es zeigen:
- Figur 1: eine erfindungsgemäße Vorrichtung zur Stimulation der oberflächlichen und tieferen Gewebsneubildung bei großflächigen und tiefen Gewebedefekten, mit einem horizontal und vertial wirkenden Distraktor in perspektivischer Darstellung;
- Figuren 2 bis 4: Distraktorsegmente des Distraktors nach der Erfindung mit unterschiedlichen Dornformen in steckbarer Modulbauweise und
- Figur 5: eine weitere Ausführung des Distraktors zur Stimulation der Neubildung von Gewebe.

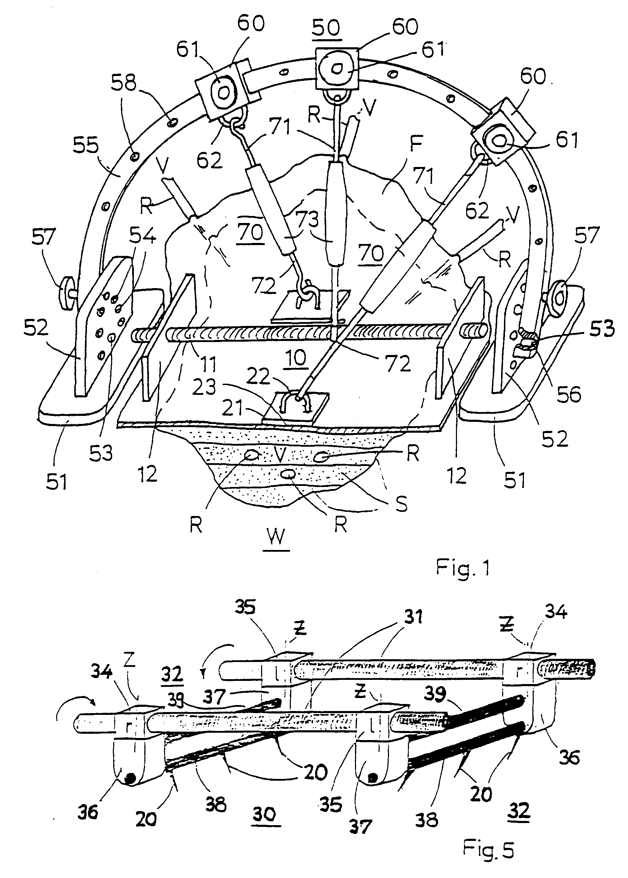

Figur 1 zeigt die grundsätzliche Anordnung der Vorrichtung zur Neubildung von Gewebe bei einer großflächigen, tiefen Wunde W, die mit Polyvinylschaumstofflagen S ausgekleidet ist, in die Drainagerohre R eingebracht sind, welche mit einem nicht dargestellten Vakuumsystem in Wirkverbindung stehen. Die Polyvinylschaumstofflagen S sind großflächig durch eine die Wundränder weitläufig abdeckende, transparente, wasserdampfpermeable aber bakterienundurchlässige Polyurethanabdeckfolie F abgedeckt, die nach Anlegen eines Vakuums V über die Drainagerohre R vom atmosphärischen Luftdruck an den Wundrändern dichtschließend angedrückt wird und so die Wunde versiegelt.

Ferner ist ein vertikal wirkender Distraktor 50 vorgesehen. Hierzu sind auf der Abdeckfolie F ein oder mehrere Zuganker angeordnet, die aus auf der Abdeckschicht flächig aufliegenden beliebig zuschneidbaren Platten 21 bestehen, an denen einerseits Ösen 22 etwa senkrecht stehend angebracht sind und die auf der abgewandten Seite mit einer Adhesivschicht 23 versehen sind, mittels der die Platten 21 flächig aufliegend auf der Abdeckfolie F durch Aufkleben befestigt sind.

Alternativ oder zusätzlich zu den auf der Abdeckfolie F aufgeklebten Platten 21 können an den Polyvinylschaumstofflagen S Anker angeordnet sein, die aus horizontal in die Polyvinylschaumstofflagen S eingebetteten Stäben oder unter den Polyvinylschaumstofflagen S angeordneten Gittern oder dergleichen bestehen.

An jeweils gegenüberliegenden Bereichen der Wundränder sind den Distraktor 50 abstützende Halteglieder 51 vorgesehen, von denen jedes mittig ein Stützlager 52 aufweist, in dem zentrisch eine zylindrische Bohrung 53 eingebracht ist. In einem Abstand von der Bohrung 53 sind Gewindebohrungen 54 im Stützlager 52 eingebracht. Die Halteglieder werden vorzugsweise mittels Manschetten, Longuetten oder vorgefertigten Schalen am Körper fixiert, wobei die Halteglieder auf den Manschetten, Longuetten oder Schalen entsprechend den Erfordernissen der zu behandelnden Verletzung aufgeklebt sind.

Den Stützlagern 52 ist ein halbkreisförmiger Bügel 55 zugeordnet, der mittels an seinen Enden angebrachten, nach innen weisenden Bolzen 56 - von denen nur der rechte gezeigt ist, in den Bohrungen 53 der Lager 52 ruht. Der Bügel 55 weist an seinen Enden je in einem Abstand vom Bolzen 56 angeordnet jeweils eine Rändelschraube 57 auf, mit denen der Bügel 55 an den Lagern 52 durch Einschrauben der Rändelschrauben 57 in die Gewindebohrungen 54 in unterschiedlicher Winkellage befestigt werden kann. Der Bügel 55 weist ferner eine Reihe axialer Gewindebohrungen 58 auf. Auf dem Bügel 55 sind Gleitsteine 60 verschieblich gelagert, die mittig jeweils eine Rändelschraube 61 aufweisen, mit der der Gleitstein 60 durch Einschrauben der Rändelschraube 61 in einer der Gewindebohrungen 58 des Bügels 55 festlegbar ist. Ferner weist jeder Gleitstein 60 an seiner der Wunde zugekehrten Seite eine Öse 62 auf.

In die Ösen 62 der Gleitsteine 60 und in die Ösen 22 der Zuganker 21 werden jeweils Haken 71, 72 eines Spannschlosses 70 eingehängt. Sind Anker in oder unter den Polyvinylschaumstofflagen S angeordnet, so greifen die Spannschlösser vorzugsweise über ein Zugseil an diesen Ankern an. Das Zugseil wird abgedichtet durch die Polyvinylschaumstofflagen hindurchgeführt. Zum Zwecke der Einstellung der Zugkraft sind die Haken 71 und 72 des Spannschlosses 70 je von einem ein Rechts- und Linksgewinde aufweisenden Spannschloßmittelteil 73 umfaßt.

Entsprechend den Gegebenheiten der Wunde des Verletzten kann der Bügel 55 um die Bolzen 56 geschwenkt und mitttels der Rändelschrauben 57 auf den Stützlagern 52 befestigt werden. Die Gleitsteine 60 können auf dem Bügel 55 so angeordnet werden, daß eine für die Steuerung der Stimulation der Neubildung des Gewebes erforderliche Richtung der Zugkraft erhalten wird, und über die Spannschlösser 70 kann dann die gewünschte Zugkraft gezielt auf einen Wundbereich oder die ganze Wunde ausgeübt werden, um so eine Revitalisierung insbesondere tiefer liegender Geweberegionen wirksam zu steuern.

An jeweils gegenüberliegenden Seiten der Wundränder kann nach dem Entfernen der Vakuumversiegelung ein horizontal wirkender Distraktor 10 angeordnet werden, der zwei Distraktorsegmente 12 umfaßt, die mittels einer Gewindespindel 11 entsprechend der Steigung der Gewindespinel aufeinander zu- oder voneinanderweg bewegbar sind. Die Distraktorsegmente 12 weisen Dorne 20 auf, die im Bereich der Wundränder in die Cutis eingesetzt werden.

Ein Spannschloß 70, der mit seinem einen Haken 71 in die Öse 62 eines Gleitsteines 60 eingehängt ist, wird mit seinem anderen Haken 72 in die Gewindespindel 11 des horizontal wirkenden Distraktors 10 eingehängt. Über das spannschloß 70 wird eine mittels des Spannschloßmittelteils 73 einstellbare vertikale Zugkraft auf die Gewindespindel 11 und damit die Distraktorsegmente 12 ausgeübt. Zusätzlich zu der über die Gewindespindel 11 erzeugten horizontal in der Ebene der Wundfläche wirkenden Zugkraftkomponente ergibt sich dadurch eine vertikal auf die Wundränder wirkende Zugkraftkomponente. Diese vertikal wirkende Zugkraftkomponente begünstigt die Gewebsneubildung im Bereich der Wundränder.

Entsprechend den Erfordernissen der Wunde können auch mehrere vertikal und/oder horizontal wirkende Distraktoren modulweise oder in Form eines Universaldistraktors mit einer Vielzahl von Zugankern 21 vorgesehen werden, um über die zugeordneten Spannschlösser 70 gezielt in jeder gewünschten Richtung Zugspannungen auf die Wunde ausüben zu können.

Die Halteglieder 51 sind also Widerlager des vertikal wirkenden Distraktors 50 zur Übertragung der Zugkräfte des Distraktors 50. Zur Anpassung an die sphärischen Gegebenheiten im an den Gewebsdefekt angrenzenden Gebiet können zwischen den Haltegliedern 51 und unverletztem Gewebe formbare, die Distraktions-Vorrichtung fixierende Unterlagen vorgesehen werden, die die gleichmäßige Verteilung der dort als Druckkräfte wirkenden Zugkräfte ermöglicht. Hierbei können die Halteglieder 51 der Vorrichtung auf herkömmlichen Gips-, auf Kunststoffbandagen oder auf während der Verarbeitung in die gewünschte Form bringbare, stoß- und bruchfeste, hypoallergene Polyvinylalkoholprodukte angebracht werden.

Es ist ohne weiteres ersichtlich, daß die vertikal wirkenden Zugkräfte nicht notwendig über Halteglieder an dem Körper des Patienten abgestützt werden müssen. Alternativ hierzu kann der vertikal wirkende Distraktor 50, an welchem die Spannschlösser 70 eingehängt werden, an einem Galgen oder einer ähnlichen am Bett des Patienten montierten Vorrichtung befestigt sein.

Beim horizontal wirkenden Distraktor 10 wird über die Gewindespindel 11 die die Distraktorsegemente 12 aufeinander zu ziehende Distraktionskraft aufgebracht, mit deren Hilfe das Zusammenwachsen des Coriums gesteuert wird.

Hierbei ergänzen sich die über den horizontal wirkenden Distraktor 10 und den vertikal wirkenden Distraktor 50 aufgebrachten Zugkräfte.

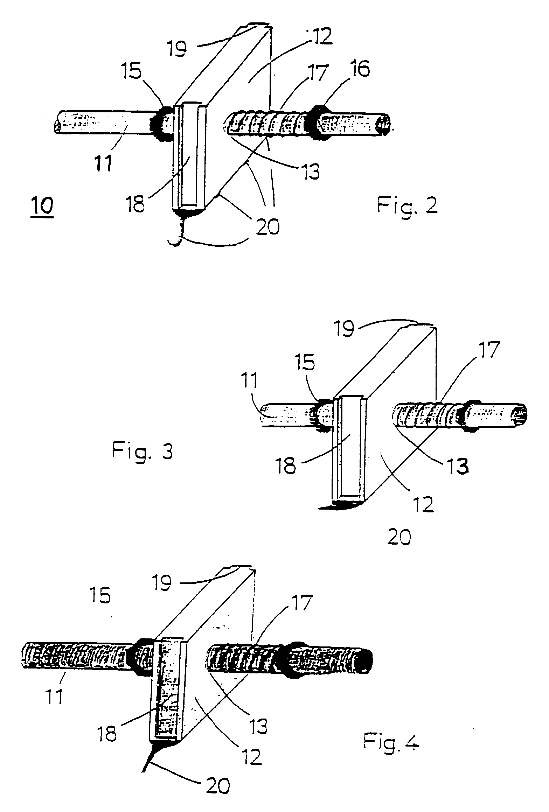

Wie aus den Figuren 2, 3 und 4 ersichtlich ist, weisen die Distraktorsegmente 12 des Distraktors 10 eine zentrale Bohrung 13 auf, in der die Gewindespindel 11 geführt ist. Auf der zur Wunde hingekehrten Seite des Distraktorsegmentes 12 ist jeweils eine als Funktionsanschlag dienende Gewindemutter 15 auf der Gewindespindel vorgesehen. Auf der der Wunde abgewandten Seite der Distraktorsegmente 12 ist auf der Gewindespindel 11 eine Druckfeder 17 angeordnet, die von einer zugeordneten, auf der Gewindespindel 11 verstellbar gelagerten Gewindemutter 16 derart beaufschlagt ist, daß die Federkraft, mit der die Distraktorsegmente 12 beaufschlagt sind, einstellbar ist.

Die Distraktorsegmente 12 und 13 weisen an ihren Seitenkanten jeweils auf der einen Seite eine Nut 18 und auf der dieser gegenüberliegenden Seite eine Feder 19 auf. Die der Wunde zugekehrte Unterkante dagegen trägt mehrere nadelförmige Dorne 20. Die Ausgestaltung der Dorne 20 ist vom Einsatzgebiet der Körperoberfläche abhängig. So zeigt die Figur 2 hakenförmige, die Figur 3 krallenförmige und die Figur 4 in ca 20° in Richtung zur Wunde angestellte Dorne. Die Dorne werden im Bereich der Wundränder in die Cutis eingelassen.

Diese Ausbildung der Distraktorsegmente 12 erlaubt den modularen Aufbau eines der individuellen Wundausdehnung angepaßten Distraktors 10 durch Aneinanderfügen von mehreren Distraktorsegmenten 12, indem diese durch Einfügen der Nut 18 des einen Distraktorsegmentes in die Feder 19 des benachbarten Distraktorsegmentes miteinander verbunden werden.

Das in Figur 5 gezeigte weitere Ausführungsbeispiel eines Distraktors 30 umfaßt zwei mit zwei Gewindespindeln 31 aufeinander zu- und voneinanderweg bewegbare Distraktorsegmente 32. Die Gewindespindeln 31 weisen jeweils einen Endes ein Rechts- und anderen Endes ein Linksgewinde auf. Wie die Figur 5 ferner zeigt, stehen die der Veränderung der Zugkraft dienenden Rechts- und Linksgewinde der Gewindespindel 31 jeweils mit korrespondierenden Gewindebohrungen in Sockeln 34 und 35 in Wirkverbindung, denen je um die vertikalen Achsen Z schwenkbar gelagerte Drehsegmente 36 und 37 zugeordnet sind. Die Drehsegmente 36 und 37 sind mittels zweier biegbarer Verstrebungen 38 und 39 kraft- und formschlüssig verbunden. Jede Verstrebung 38 trägt eine um etwa 20° zur Z-Achse zur Wunde hin ausgerichtete Reihe von Dornen 20.

Alle Bauteile der Vorrichtung sind aus Titan oder Osteosynthesestahl hergestellt. Es können aber auch Kunststoffe oder Kohlefaserverbundstoffe, die den Erfordernissen gerecht werden, zur Anwendung gelangen.

Durch die beschriebene Vorrichtung zur Stimulation der Neubildung von Gewebe bei großflächigen und tiefen Gewebedefekten wird die Mortalität sowie die Komplikationshäufigkeit wesentlich reduziert.

Darüber hinaus erlaubt die beschriebene Vorrichtung nach der Erfindung die Narbenbildung zu steuern und zu verringern.

Schließlich wird durch konsequente Anwendung der beschriebenen Vorrichtung die gesamte Genesungsdauer verkürzt.

## Patentansprüche

1. Vorrichtung zur Stimulation der Neubildung von Gewebe bei großflächigen und tiefen Wunden, mit einer die Wunde und deren Wundränder abdichtend überdeckenden Kunststofffolie (F), dadurch gekennzeichnet, daß an der Kunstofffolie im Bereich des Wundfeldes wenigstens ein Zuganker (21) angeordnet ist, der über Stellmittel (70) mit einer das Wundfeld überspannenden Halterung (50) verbunden ist und eine Zugkraft mit einer zur Ebene des Wundfeldes senkrechten Komponente auf die Kunststofffolie (F) ausüben kann.

2. Vorrichtung nach Anspruch 1, gekennzeichnet durch wenigstens eine offenporige Schaumstoffschicht (S), die in das Wundfeld einlegbar ist, durch wenigstens einen in die Schaumstoffschicht (S) eingebrachten Drainage-Schlauch (R) und durch eine an den Drainage-Schlauch (R) anschließbare Absaugung, wobei die Kunststofffolie (F) die Schaumstoffschicht (S) überdeckt.

3. Vorrichtung nach Anspruch 1 oder 2, dadurch gekennzeichnet, daß der/die Zuganker (21) flächig mit der Kunststofffolie (F) verbunden sind.

4. Vorrichtung nach Anspruch 3, dadurch gekennzeichnet, daß der Zuganker (21) aus einer ebenen Platte besteht, auf der eine senkrechte Öse (22) angebracht ist und die mit einer Adhäsivschicht auf die Kunststofffolie (F) geklebt ist.

5. Vorrichtung nach Anspruch 2, dadurch gekennzeichnet, daß der wenigstens eine Zuganker (21) an einer der Schaumstoffschichten (S) angreift und daß die Stellmittel (70) abgedichtet durch die Kunststofffolie (F) hindurchgeführt sind.

6. Vorrichtung nach Anspruch 5, dadurch gekennzeichnet, daß die Stellmittel (70) über ein Zugseil an der Schaumstoffschicht (S) angreifen, wobei das Zugseil an dem in die Schaumstoffschicht (S) eingebetteten oder unter der Schaumstoffschicht (S) liegenden quer zur Zugrichtung des Zugseiles angeordneten Zuganker angreift oder in die Schaumstoffschicht (S) eingeknotet ist.

7. Vorrichtung nach Anspruch 5 oder 6, dadurch gekennzeichnet, daß die Durchtrittsstelle der Stellmittel bzw. des Zugseiles durch die Kunststofffolie (F) mittels einer Hydrogel-Masse abgedichtet ist.

8. Vorrichtung zur Stimmulation der Neubildung von Gewebe bei großflächigen und tiefen Wunden, mit wenigstens einem nahe den Wundrändern in die Cutis eingreifenden Element (12, 32) dadurch gekennzeichnet, daß das Element (12, 32) über Stellmittel (70) mit einer die Wunde überspannenden Halterung (50) verbunden ist, wobei die Stellmittel (70) auf das Element (12, 32) eine Kraft mit einer zur Wundfläche senkrechten Komponente ausüben können.

9. Vorrichtung nach Anspruch 8, dadurch gekennzeichnet, daß an einander gegenüberliegenden Wundrändern zwei Elemente als Distraktorsegmente (12, 32) eines Distraktors (10) angeordnet sind, die aufeinander zu- und voneinanderweg bewegbar sind.

10. Vorrichtung nach Anspruch 9, dadurch gekennzeichnet, daß die Distraktorsegmente (12, 32) über ein Gewinde (11, 31) gegeneinander bewegbar sind.

11. Vorrichtung nach einem der Ansprüche 8 bis 10, dadurch gekennzeichnet, daß die Distraktorsegmente (12, 32) mit Dornen (20) in die Cutis eingreifen.

12. Vorrichtung nach Anspruch 10, dadurch gekennzeichnet, daß die Distraktorsegmente (12) jeweils wenigstens eine zylindrische Bohrung (13) aufweisen, in der eine Gewindestange (11) längs verschieblich geführt ist, auf der eine als Anschlag für das Distraktorsegment (12) dienende Gewindemutter (15) angeordnet ist, während auf der gegenüberliegenden Seite eine weitere Gewindemutter (16) das Distraktorsegment (12) über eine Druckfeder (17) kraftbeaufschlagt.

13. Vorrichtung nach Anspruch 10, dadurch gekennzeichnet, daß die Distraktorsegmente (12) jeweils wenigstens eine Gewindebohrung aufweisen und daß eine die Distraktorsegmente (12) verbindende Gewindestange mit jeweils die Relativbewegung der Distraktorsegmente (12) bewirkenden Links- und Rechtsgewinden in die Gewindebohrungen eingreift.

14. Vorrichtung nach Anspruch 13, dadurch gekennzeichnet, daß jedes Distraktorsegment (32) zwei Drehsegmente (36, 37) umfaßt, die über Verstrebungen (38, 39) verbunden sind, und daß die Drehsegmente (36, 37) um eine Z-Achse schwenkbar in Sockeln (34, 35) gelagert sind, die die Gewindebohrungen aufweisen.

15. Vorrichtung nach einem der Ansprüche 11 bis 14, dadurch gekennzeichnet, daß die Distraktorsegmente (12, 32) an ihrer unteren Seite eine Reihe von Dornen (20) aufweisen.

16. Vorrichtung nach einem der Ansprüche 9 bis 15, dadurch gekennzeichnet, daß jeweils zwei Distraktorsegmente (12) mit ihrer Gewindeverstellung ein Distraktormodul (10) bilden.

17. Vorrichtung nach Anspruch 16, dadurch gekennzeichnet, daß die Distraktorsegmente (12) durch eine jeweils an ihren Stirnseiten ausgebildeten Nut- und Federverbindung (18, 19) modulartig zusammensetzbar sind.

18. Vorrichtung nach Anspruch 1 oder 8, dadurch gekennzeichnet, daß die Halterung (50) einen bogenförmigen Bügel (55) aufweist, der beidendig abstützbar ist.

19. Vorrichtung nach Anspruch 18, dadurch gekennzeichnet, daß der Bügel (55) mit seinen Enden schwenkbar und feststellbar in Stützlagern (52) gelagert ist.

20. Vorrichtung nach Anspruch 18 oder 19, dadurch gekennzeichnet, daß auf dem Bügel (55) verschieblich und feststellbar Gleitsteine (16) angeordnet sind, die zum Einhängen der Stellmittel (70) dienen.

21. Vorrichtung nach Anspruch 1 oder 8, dadurch gekennzeichnet, daß die Stellmittel als längenverstellbare Spannschlösser (70) ausgebildet sind.

## Claims

1. Device for stimulating the regeneration of tissue in the case of large-area and deep wounds, having a plastics film (F) which covers the wound and its wound edges as a seal, characterized in that there is disposed on the plastics film in the region of the wound area at least one traction anchor (21) which is joined via adjustment means (70) to a frame (50) which covers the wound area and can exert a traction force having a component perpendicular to the plane of the wound area on the plastics film (F).

2. Device according to Claim 1, characterized by at least one open-pore foam-material layer (S) which can be inserted into the wound area, by at least one drainage hose (R) introduced into the foam-material layer (S) and by a suction system which can be connected to the drainage hose (F), the plastics film (F) covering the foam-material layer (S).

3. Device according to Claim 1 or 2, characterized in that the traction anchor(s) (21) is/are joined two-dimensionally to the plastics film (F).

4. Device according to Claim 3, characterized in that the traction anchor (21) comprises a flat plate on which a vertical eyelet (22) is provided and which is glued to the plastics film (F) by an adhesive layer.

5. Device according to Claim 2, characterized in that the at least one traction anchor (21) acts on one of the foam-material layers (S) and in that the adjustment means (70) are passed through the plastics film (F) in a sealed manner.

6. Device according to Claim 5, characterized in that the adjustment means (70) act on the foam-material layer (S) via a traction cable, the traction cable acting on a traction anchor which is disposed transversely to the traction direction of the traction cable and embedded in the foam-material layer (S) or situated underneath the foam-material layer (S) or is tied into the foam-material layer (S).

7. Device according to Claim 5 or 6, characterized in that the point at which the adjustment means or the traction cable passes through the plastics film (7) is sealed by means of a hydrogel compound.

8. Device for stimulating the regeneration of tissue in the case of large-area and deep wounds, having at least one element (12, 32) which bites into the cutis near the wound edges, characterized in that the element (12, 32) is joined to a frame (50) covering the wound via adjustment means (70), the adjustment means (70) being able to exert a force having a component perpendicular to the wound area on the element (12, 32).

9. Device according to Claim 8, characterized in that there are disposed at mutually opposite wound edges two elements as distractor segments (12, 32) of a distractor (10), which elements can be moved towards and away from one another.

10. Device according to Claim 9, characterized in that the distractor segments (12, 32) can be moved with respect to one another by means of a thread (11, 31).

11. Device according to one of Claims 8 to 10, characterized in that the distractor segments (12, 32) bite into the cutis by means of spikes (20).

12. Device according to Claim 10, characterized in that the distractor segments (12) each have at least one cylindrical bore (13) into which a threaded rod (11) is passed in a longitudinally displaceable manner, on which rod (11) a threaded nut (15) is disposed which acts as a stop for the distractor segment (12), while, on the opposite side, a further threaded nut (16) applies force to the distractor segment (12) by means of a tension spring (17).

13. Device according to Claim 10, characterized in that the distractor segments (12) each have at least one threaded bore and in that a threaded rod which joins the distractor segments (12) and which has left-hand and right-hand threads which each effect the relative movement of the distractor segments (12) engages in the threaded bores.

14. Device according to Claim 13, characterized in that each distractor segment (32) comprises two rotation segments (36, 37) which are joined by means of struts (38, 39), and in that the rotation segments (36, 37) are mounted in supports (34, 35) so as to be able to swivel around a Z-axis, which supports (34, 35) comprise the threaded bores.

15. Device according to one of Claims 11 to 14, characterized in that the distractor segments (12, 32) have a series of spikes (20) on their underside.

16. Device according to one of Claims 9 to 15, characterized in that two distractor segments (12) each form a distractor module (10) with their threaded adjustments.

17. Device according to Claim 16, characterized in that the distractor segments (12) can be assembled in modular fashion by means of a groove and tongue joint (18, 19) formed in each case on their end faces.

18. Device according to Claim 1 or 8, characterized in that the frame (50) has an arc-shaped bracket (55) which can be supported at both ends.

19. Device according to Claim 18, characterized in that the ends of the bracket (55) are mounted in supporting mounts (52) in a swivellable and lockable manner.

20. Device according to Claim 18 or 19, characterized in that there are disposed on the bracket (55) in a displaceable and lockable manner sliding blocks (61) which serve to secure the adjustment means (70).

21. Device according to claim 1 or 8, characterized in that the adjustment means are designed as longitudinally adjustable turnbuckles (70).

## Revendications

1. Dispositif pour stimuler la formation de nouveaux tissus dans le cas de blessures de grande surface et profondes, avec une feuille de matière plastique (F), recouvrant de façon étanche la blessure et ses bords,
caractérisé en ce qu'
on dispose sur la feuille de matière plastique dans la région du champ de la blessure au moins une armature de traction (21), qui est reliée par l'intermédiaire d'un moyen de réglage (70) à un support (50), qui surplombe le champ de la blessure et qui peut exercer une force de traction par une composante perpendiculaire au plan de la blessure sur la feuille de matière plastique (F).

2. Dispositif selon la revendication 1,
caractérisé par
au moins une couche d'un produit alvéolaire à pores ouverts (S), qui peut être posée sur le champ de la blessure, par au moins un tuyau de drainage (R), que l'on peut mettre dans la couche de matière alvéolaire, et par une aspiration, que l'on peut raccorder au tuyau de drainage (R), la feuille de matière plastique (F) recouvrant la couche de matière alvéolaire (S).

3. Dispositif selon la revendication 1 ou 2,
caractérisé en ce que
la ou les armatures de traction (21) sont reliées à plat à la feuille de matière plastique (F).

4. Dispositif selon la revendication 3,
caractérisé en ce que
l'armature de traction (21) consiste en une plaque plane, sur laquelle est mis un oeillet perpendiculaire (22) et qui est collée à une couche adhésive sur la feuille de matière plastique (F).

5. Dispositif selon la revendication 2,
caractérisé en ce que
- l'une au moins des armatures de traction (21) vient en prise sur l'une des couches de matière plastique (S) et
- on fait passer à travers la feuille de matière plastique (F) de façon étanche les moyens de réglage (70).

6. Dispositif selon la revendication 5,
caractérisé en ce que
les moyens de réglage (70) viennent en prise au moyen d'un câble de traction sur la couche de matière alvéolaire (S), le câble de traction étant enrobé dans la couche de matière alvéolaire (S), ou se trouvant en dessous de la couche de matière alvéolaire (S) en étant disposé perpendiculairement à la direction de traction du câble de traction ou étant noué dans la couche de matière alvéolaire (S).

7. Dispositif selon la revendication 5 ou 6,
caractérisé en ce que
le point de passage du moyen de réglage ou du câble de traction à travers la feuille de matière plastique (F) est rendu étanche au moyen d'une masse d'hydrogel.

8. Dispositif servant à stimuler la formation de nouveaux tissus dans le cas de blessures de grande surface et profondes, avec au moins un élément (12, 32) venant en prise dans le derme à proximité des bords de la blessure,
caractérisé en ce que
l'élément (12, 32) est relié par l'intermédiaire de l'organe de réglage (70) à une fixation (50) qui surplombe la blessure, l'organe de réglage (70) pouvant exercer sur l'élément (12, 32) une force avec une composante perpendiculaire à la surface de la blessure.

9. Dispositif selon la revendication 8,
caractérisé en ce qu'
on dispose sur des bords de la blessure opposés les uns aux autres deux éléments servant de segments (12, 32) d'un écarteur (10), que l'on peut déplacer l'un sur l'autre en les rapprochant ou les écartant l'un de l'autre.

10. Dispositif selon la revendication 9,
caractérisé en ce que
les segments de l'écarteur (12, 32) peuvent être déplacés au moyen d'un filetage (11, 31).

11. Dispositif selon l'une des revendications 8 à 10,
caractérisé en ce que
les segments de l'écarteur (12, 32) viennent en prise dans le derme par des crochets (20).

12. Dispositif selon la revendication 10,
caractérisé en ce que
les segments de l'écarteur (12) présentent respectivement au moins un alésage cylindrique (13), dans lequel on fait coulisser une tige filetée (11) dans le sens de la longueur, tige filetée sur laquelle est disposée un écrou fileté (15) qui sert de butée au segment de l'écarteur (12), tandis que sur le côté opposé un autre écrou fileté (16) exerce une force sur le segment de l'écarteur (12) au moyen d'un ressort de compression (17).

13. Dispositif selon la revendication 10,
caractérisé en ce que
- les segments de l'écarteur (12) présentent respectivement au moins un alésage fileté et
- une tige filetée, qui relie les segments de l'écarteur (12), vient en prise dans les alésages filetés avec respectivement des filetages à droite et à gauche, qui provoquent le mouvement relatif des segments de l'écarteur (12).

14. Dispositif selon la revendication 13,
caractérisé en ce que
- chaque segment de l'écarteur (32) comprend deux segments de rotation (36, 37), qui sont reliés au moyen de tirants (38, 39), et
- les segments de rotation (36, 37) sont montés de façon à pouvoir pivoter autour d'un axe Z dans des socles (34, 35), qui présentent les alésages filetés.

15. Dispositif selon l'une des revendications 11 à 14,
caractérisé en ce que
les segments de l'écarteur (12, 32) présentent sur leur côté inférieur une série de crochets (20).

16. Dispositif selon l'une des revendications 9 à 15,
caractérisé en ce que
respectivement deux segments de l'écarteur (12) forment par leur ajustage par filetage un module d'écarteur (10).

17. Dispositif selon la revendication 16,
caractérisé en ce que
les segments de l'écarteur (12) peuvent être réunis d'une manière modulaire par une liaison (18, 19) à rainure et ressort, constituée -respectivement sur leurs côtés frontaux.

18. Dispositif selon la revendication 1 ou 8,
caractérisé en ce que
la fixation (50) présente un étrier (55) en forme d'arceau, que l'on peut mettre en appui des deux côtés.

19. Dispositif selon la revendication 18,
caractérisé en ce que
l'étrier (55) est monté de façon à pouvoir pivoter à ses extrémités et à pouvoir être immobilisé dans des paliers d'appui (52).

20. Dispositif selon la revendication 18 ou 19,
caractérisé en ce que
sur l'étrier (55) on dispose des blocs de glissement (16), de façon à ce qu'ils puissent coulisser et être fixés sur l'étrier (55), blocs qui servent à accrocher les moyens de réglage (70).

21. Dispositif selon la revendication 1 ou 8,
caractérisé en ce que
les moyens de réglage sont constitués sous la forme de tendeurs (70), que l'on peut régler dans le sens de la longueur.
